# EUROPEAN PATENT APPLICATION

(11) **EP 1 488 745 A1**
(43) Date of publication of application: **22.12.2004**
(21) Application number: 04014289.5
(22) Date of filing: 18.06.2004
(51) Int. Cl.: A61B 17/16, A61C 8/00

(54) **Osteotome Structure**

(30) Priority: 20.06.2003 IT PS20030026
(71) Applicant: Corigliano, Massimo, 00015 Monterotondo (Roma) (IT)
(72) Inventor: Corigliano, Massimo, 00015 Monterotondo (Roma) (IT)
(74) Representative: Gustorf, Gerhard, Dipl.-Ing.

(57) **Abstract**

Osteotome structure, including an instrument (2) with a truncated tip (3) that is distally conical (4) and proximally cylindrical (5) provided on the outer edge with at least three cutting edges (7) whose extension is preferably helical, with blades (8) inclined in the direction of screwing in relation to the radial directrix, interspersed with deep grooves (9) with a "V" section in which radial holes (10, 11) are arranged at the apex and in the intermediate area for adduction of physiological solution brought in through axial channelling (12).

## Description

In the implantation of dental prostheses and in orthopaedics the instruments usually employed for creating sockets in osseous tissue for fitting inserts or prostheses are traditional osteotribes, either spiral-shaped or straight and cannon-shaped, with cuts on the longitudinal axis of the instrument.

These osteotribes remove osseous tissue by means of erasion-abrasion and for that purpose are made to rotate at high speed, which overheats the osseous tissue treated, in the area around the socket created, causing thermal stress to the point of narcotising it, and in any case having a decidedly negative effect on the prospect of bone integration of the insert or prosthesis, at least in terms of time.

The device that these osteotribes sometimes use to allow for cooling the osseous tissue during the operation, with an internal socket irrigated with physiological solution, is not an ideal way of remedying the problem.

The tip of these osteotribes also has a boring action, and consequently there is the contra-indication of drilling deeply into the bone, which is decidedly invasive.

### Aims of the invention

In this context, the main aim of this invention is to provide an innovative instrument structure suitable for making sockets in osseous tissues as accurately as possible, for the purpose of implantation of dental prostheses in general and orthopaedic ones in particular, without producing a heating effect such as to damage the osseous tissue.

Another aim of this invention is to achieve the previous aim by means of a solution concept that minimizes the invasiveness and stress of the operation.

Yet another aim of this invention is to achieve the previous aims by means of a structure that makes it possible to recover bone fragments under conditions that permit their effective reuse in procedures of implantation and of bone grafting in accordance with the principles of guided bone regeneration.

A further aim of this invention is to achieve the above aims while nevertheless attaining a simple and effective structure, safe in operation and of relatively low cost in relation to the results achieved in practice with it.

### Solution concept summary extract

These and yet other aims are all achieved with the osteotome structure presented in this invention, including an instrument (2) with a truncated tip (3) that is distally conical (4) and proximally cylindrical (5) provided on the outer edge with at least three cutting edges (7) whose extension is preferably helical, with blades (8) inclined in the direction of screwing in relation to the radial directrix, interspersed with deep grooves (9) with a "V" section in which radial holes (10, 11) are provided at the apex and in the intermediate area for adduction of physiological solution brought in through axial channelling (12).

### Identification of the attached drawings

Further characteristics and advantages of the osteotome structure according to this invention will become clearer from the detailed description that follows of a preferred but not exclusive form of production of it, presented purely for exemplary purposes and without limitation in the sole table of drawings attached, in which:
Figure 1 shows a partially cut away lateral view with details shown in outline of an osteotome structure according to this invention;
Figure 2 shows a frontal view of the tip of the structure.

### Static description of the implementation example

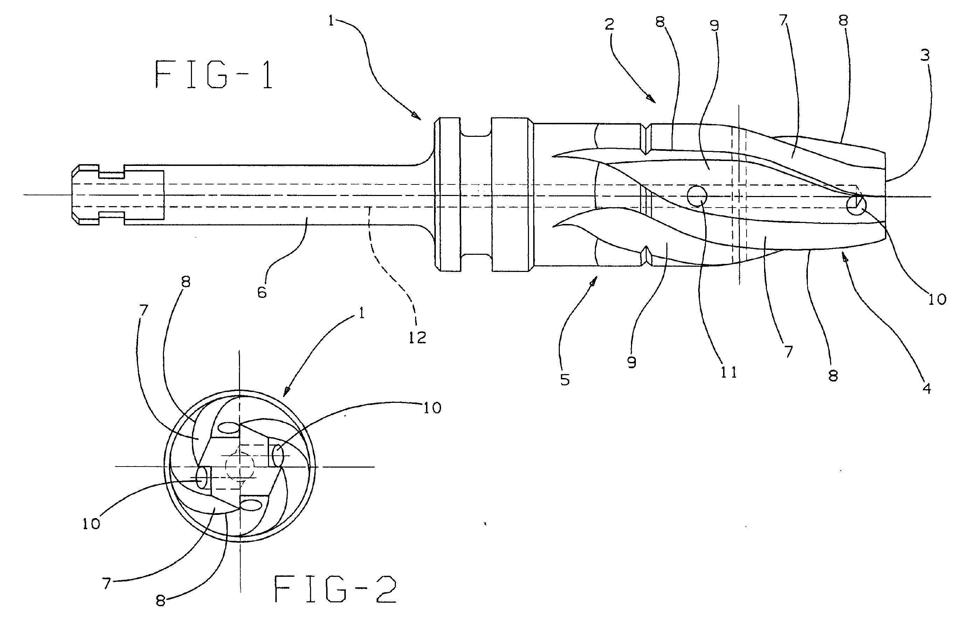

With reference to these figures, and especially figure 1, no.1 shows an overall view of an osteotome structure according to this invention, including instrument 2 with tip 3, flat or arched but in any case truncated, with a conical distal portion 4 connected to a cylindrical proximal portion 5.

The instrument 2 is attached to a shaft 6 that can be adapted to ordinary drills or motors for intraosseus implantation and has at least three cutting ridges 7 on the outer edge, whose extension is preferably helical, with their respective blades 8 inclined in the direction of screwing in relation to the radial directrix and sharpened to the maximum.

The cutting ridges 7 are interspersed with marked grooves 9 with a "V" section, within which there are apical radial holes 10 and intermediate radial holes 11 for adduction of physiological solution, brought through axial channelling 12 for the functions that will be specified below.

### Dynamic description of the implementation example

Having thus finished the static description of a preferred example of production of the osteotome structure according to this invention, we will now move on to the dynamic one, dealing with the way it works:
by means of its shaft 6 the instrument 2 can be connected to any intraosseus implantation motor or drill, including ones that work manually, and can be used to make sockets in osseous tissues in the implantation of dental prostheses and in orthopaedics like ordinary osteotribes;
unlike the latter, the instrument 2 does not remove osseous tissue by erasion or abrasion, but rather by cutting, so that it can and must be used at low rotational speeds (max 100 rpm ) thus avoiding overheating and necrosis of the living bone.

In this way, thanks to the structural characteristics of the instrument 2 and to the low speed in use, the survival of the cellular components of the osseous tissue removed is ensured, and especially those nearby, which are usually killed by the use of osteotribes with the known technique.

On the other hand the instrument 2 for bone excavation according to this invention is not similar to osteotribes. It is in fact a true osteotome and is therefore a remedial instrument with which the socket can be made without the need for intermediate steps.

It should be noted that the instrument 2 for bone excavation according to this invention can be used only after carrying out a prior sounding of the bone width with a preliminary osteotribe probe.

The flat and in any case truncated tip 3 of the instrument 2, or rather the absence of cutting at the tip contributes to reducing the invasiveness of the operations in which it is used;
the flat or rounded tip enables the instrument 2 according to this invention to be even less invasive during osteotomy work, especially as regards anatomical structures at risk, such as the antrum of Highmore or the mandibular alveolar nerve that might otherwise be damaged by cutting or invasive instruments.

Furthermore, the structure of the cutting ridges 7 and of the marked grooves 9 interposed between them, as well as the shape of the blade 8, make it possible to recover the osseous tissue within the grooves 9 interposed between blade 8 and blade 8, where the "V" shape of the grooves 9 fosters the accumulation of live osseous tissue.

As it is not thermally damaged, this osseous tissue, recovered with the instrument 2 according to this invention, can be reused in procedures of implantation and of bone grafting in accordance with the principles of guided bone regeneration.

In this context, close to the tip 3, between the recovery grooves 9, there are the apical radial holes 10 and the intermediate ones 11 communicating with the central channel 12; the holes 10 and 11 in question achieve the washing of the instrument 2 during use through adduction of physiological solution; they are necessary to ensure the cleansing of the blades 8 and the outflow of the bone chips as the instrument moves forward in the newly formed socket.

Alternatively the central channel 12 can emerge at the tip 3 to increase the volume of liquid, also for cooling purposes, but in reality the central hole is not very efficient for cooling, because, as the instrument advances, not enough liquid manages to gush out, as the hole is blocked by the osseous tissue that is in contact with the tip; moreover the greater part of the heat develops along the walls of the instrument 2 and not at the tip. However, without prejudice to the fact that the primary action of draining and cooling is carried out by the lateral holes, as a certain cooling action by contact can be attributed to the central hole, it may usefully be provided;
in this context further radial holes for liquid adduction placed along the extension of the instrument 2 may be more useful.

However, the use of the instrument 2 according to this invention, whether accompanied by a cooling action with physiological solution irrigation, or carried out dry, does not in any case pose the risk of overheating the osseous tissue during the operation thanks to the great cutting efficiency of the blades and the low speed in use.

When at the end the instrument 2 has correctly shaped the socket, it rotates freely without damaging the receiving tissue.

### Implementation alternatives

It is clear that in other alternative forms of implementation, while still falling within the concept of innovation implied by the production example explained above and claimed below, the osteotome structure according to this invention could be produced with technical and structural equivalents, or accompanied by supplementary devices, just as all the shapes of the related constituent parts can be varied to suit the purpose; in particular:
the cutting ridges and the related blades can be provided in any number suited to the purpose.

It can be used as a simple remedial instrument or at the same time as a bone recovery tool.

The blades of the instrument can be vertical straight or inclined according to the pitch of the *fixture* in question, thus being equally inclined at the assembly spiral, and according to the speed of screwing of the implant.

The pitch of the helixes that are formed by inclining the blades can be varied according to the size of the bone chips that one wishes to recover. The straighter they are the greater the size and quantity of bone slivers.

Reference notches can be cut in the outer edge of the instrument to check the depth reached during socket preparation.

### Advantages of the invention

As seems self-evident from the detailed description above of a preferred example of implementation, the osteotome structure according to this invention offers advantages corresponding to the set aims, and also others.

it is in fact a simple and effective tool for making sockets, in particular in the implantation of dental prostheses and in general in orthopaedic surgery, reducing to a minimum the stress for osseous tissues close to those that are excavated, thus greatly fostering the subsequent bone integration and connection of the implants or prosthesis fitted into the sockets made, at the same time permitting the recovery of osseous tissues excavated for any reuse requirement in bone regeneration treatment and in any case for whatever the need may be.

## Claims

1. Osteotome structure **characterised by** the fact of including an instrument (2) with a truncated tip (3) that is distally conical (4) and proximally cylindrical (5) provided on the outer edge with at least three cutting edges (7) whose extension is preferably helical, with blades (8) inclined in the direction of screwing in relation to the radial directrix, interspersed with deep grooves (9) with a "V" section in which radial holes (10, 11) are provided for adduction of physiological solution brought in through axial channelling (12).

2. Osteotome structure according to the previous claim, **characterised by** the fact that the said grooves (9) have a "V" section.

3. Osteotome structure according to the previous claim, **characterised by** the fact that within these grooves (9) there are apical radial holes (10) for adduction of physiological solution brought through axial channelling (12).

4. Osteotome structure according to the previous claim, **characterised by** the fact that that within these grooves (9) there are intermediate radial holes (11) for adduction of physiological solution brought through axial channelling (12).

5. Osteotome structure according to the previous claim, **characterised by** the inclusion of a tip (3) that is flat or arched, but in any cased truncated.

6. Osteotome structure according to the previous claim, **characterised by** being equipped with a shaft (6) that is adaptable to normal motors for intraosseus implantation or drills, including manually operated ones.

7. Osteotome structure according to the previous claim, **characterised by** the fact that it is used at a maximum rotation speed of 100 rpm.

8. Osteotome structure according to the previous claim, **characterised by** the fact that it is used after carrying out a prior sounding of the bone width with a preliminary osteotribe probe.

9. Osteotome structure according to the previous claim, **characterised by** the fact that it is used for recovery of osseous tissue to be reused in procedures of bone implantation and grafting according to the principles of guided bone regeneration.

10. Osteotome structure according to the previous claim, **characterised by** the fact that the said central channel (12) emerges on the said tip (3).

11. Osteotome structure according to the previous claim, **characterised by** the fact that has further radial holes for addition of liquid placed along the extension of the said instrument (2).

12. Osteotome structure according to the previous claim, **characterised by** the fact that the said cutting ridges (7) with their related blades (8) are provided in any number suited to the purpose.

13. Osteotome structure according to the previous claim, **characterised by** the fact that the said blades (8) of the said instrument (2) are alternatively supplied vertical, straight or inclined, depending on the pitch of the *fixture* in question and the speed of screwing of the implant.

14. Osteotome structure according to the previous claim, **characterised by** the fact that the pitch of the helixes that form when the said blades are inclined is varied on the basis of the size of the bone chips that one wishes to recover, and the straighter they are the greater the quantity of bone slivers.

15. Osteotome structure according to the previous claim, **characterised by** the fact that reference notches are cut in the outer edge of the said instrument (2) to check the depth reached during socket preparation.
